(19) **Europäisches Patentamt**
**European Patent Office**
**Office européen des brevets**

(11) Veröffentlichungsnummer: **0 150 719**
**B1**

(12) # EUROPÄISCHE PATENTSCHRIFT

(45) Veröffentlichungstag der Patentschrift: **15.10.86**

(51) Int. Cl.⁴: **C 07 C 149/32,** A 61 K 31/60

(21) Anmeldenummer: **85100116.4**

(22) Anmeldetag: **07.01.85**

(54) **Substituierte Salicylsäureamide, Verfahren zu ihrer Herstellung sowie ihre Verwendung als Arzneimittel.**

(30) Priorität: **20.01.84 DE 3401950**

(43) Veröffentlichungstag der Anmeldung: **07.08.85 Patentblatt 85/32**

(45) Bekanntmachung des Hinweises auf die Patenterteilung: **15.10.86 Patentblatt 86/42**

(84) Benannte Vertragsstaaten: **BE CH DE FR GB IT LI NL**

(56) Entgegenhaltungen:
**EP-A-0 119 446**
**DE-A-2 610 837**
**US-A-3 914 418**

(73) Patentinhaber: **BAYER AG, Konzernverwaltung RP Patentabteilung, D-5090 Leverkusen 1 Bayerwerk (DE)**

(72) Erfinder: **Andrews, Peter, Dr., Gellertweg 2, D-5600 Wuppertal 1 (DE)**
Erfinder: **Böshagen, Horst, Dr., Wiesenstrasse 4, D-5657 Haan (DE)**
Erfinder: **Kölling, Heinrich, Dr., Adlerstrasse 15, D-5657 Haan (DE)**

**EP 0 150 719 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue substituierte Salicylsäureamide, Verfahren zu ihrer Herstellung und die Verwendung als Arzneimittel, insbesondere als Anthelmintika.

Es ist bereits bekannt, daß substituierte Salicylsäureamide eine anthelmintische Wirkung aufweisen (siehe dazu u.a. DE—OS—2 610 837 und US—Patent 3 914 418).

Es wurden die neuen substituierten Salicylsäureamide der Formel (I) gefunden

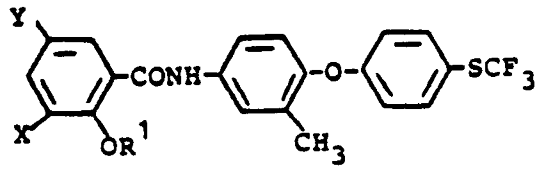

(I)

X und Y für gleiche oder verschiedene Halogene und
$R^1$ für Wasserstoff oder den Acetylrest steht.

Weiterhin wurde gefunden, daß man die neuen Salicylsäureamide der Formel (I)

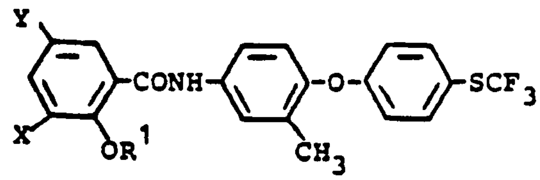

(I)

in der
X und Y für gleiche oder verschiedene Halogene und
$R^1$ für Wasserstoff oder den Acetylrest steht
erhält, wenn man die Chrloride der Saliscylsäuren oder ihrer Acetylderivate der Formel (II)

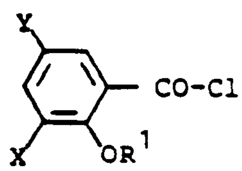

(II)

in der
$R^1$, x und Y die oben angegebene Bedeutung besitzen mit dem Amin der Formel (III)

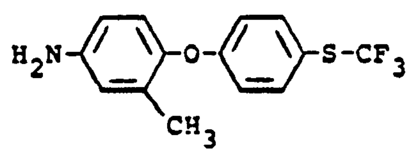

(III)

in Gegenwart eines Verdünnungsmittels, gegebenenfalls in Gegenwart eines Säurebindemittels umsetzt und gegebenenfalls den Acylrest abspaltet.

Überraschenderweise zeichnen sich die erfindungsgemäßen Salicylsäureamide im Vergleich zu anderen Anthelmintika bei guter anthelmintischer Wirkung durch ein breiteres Wirkungsspektrum aus. In Kombination mit anderen, anthelmintisch wirksamen Verbindungen, zeigen sie eine gute Kompatibilität und eine Synergistische Wirkung. Darüber hinaus durchbrechen sie die Benzimidazolresistenz bekannter Anthelmintika.

Bevorzugt sind Verbindungen der Formel (I) in welcher X und Y für Chlor, Brom, Iod insbesondere für Iod stehen und $R^1$ für Wasserstoff steht.

Verwendet man 3,5-Diiodsalicylsäurechlorid als Ausgangsmaterial der Formel (II), so kann die Reaktion mit dem Amin der Formel (III) durch das folgende Reaktionsschema wiedergegeben werden:

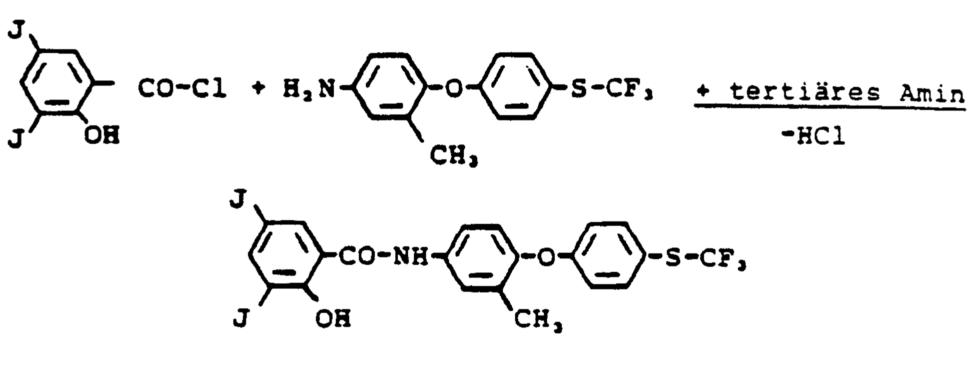

Benutzt man 3,5-Diiod-acetylsalicylsäurechlorid als Ausgangsmaterial so kann der Reaktionsablauf wie folgt formuliert werden:

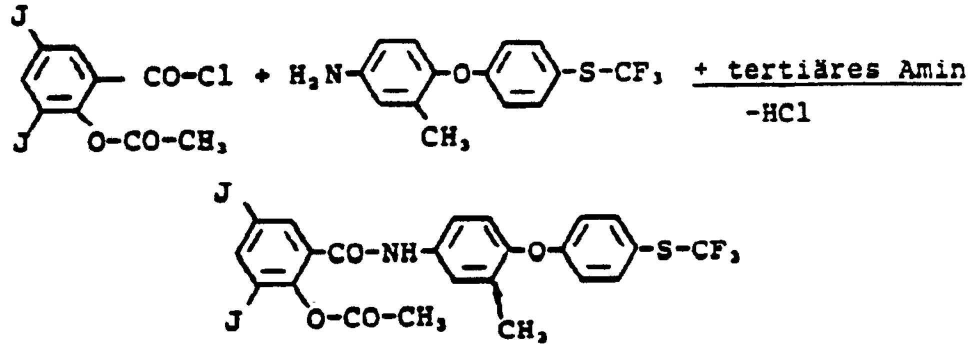


Die Acetylgruppe kann anschließend nach literaturbekannten Methoden verseift werden. Die als Ausgangsstoffe verwendeten substituierten Salicylsäurechloride der Formel (II) sind bekannt oder lassen sich nach literaturbekannten Methoden analog darstellen. Die Verbindung der Formel (III) ist bekannt (DE—OS 2 413 722).

Als Verdünnungsmittel kommen bei der Durchführung des erfindungsgemäßen Verfahrens inerte organische Lösungsmittel insbesondere Ether wie Dioxan oder Tetrahydrofuran in Frage.

Als Säurebindemittel kommen anorganische Basen wie NaOH, KOH, $Na_2CO_3$, $NaHCO_3$ oder organische Basen wie Diethylamin oder Triethylamin in Frage.

Die Reaktionstemperaturen können in einem größeren Bereich variiert werden. Im allgemeinen arbeitet man zwischen 50 und 120°C. Die Umsetzung wird bei Normaldruck durchgeführt.

Die Verseifung wird mit Säuren oder mit Basen bei Temperaturen zwischen 50 uynd 100°C, bevorzugt in wäßrigen oder wasserhaltigen Verdünnungsmitteln, insbesondere Alkohole wie Methanol oder Ethanol durchgeführt. Als Säuren seien Mineralsäuren wie Salzsäure, Schwefelsäure genannt. Als Basen seien Alkalihydroxide wie Natrium oder Kaliumhydroxid genannt.

Die neuen Verbindungen sind breit wirksam gegen human und tierpathogene Endoparasiten. Sie wirken vor allem gegen Trematoden und Nematoden, insbesondere Leberegel und Magenund Darmnematoden der Wiederkäuer. Darüber hinaus wirken sie auch gegen solche Magen- und Darmnematoden, die gegen die gebräuchlichen Benzimidazole-Anthelmintika resistent und damit nicht mehr ausreichend therapierbar sind.

Die Wirkung wurde im Tierversuch nach oraler, parenteraler und dermaler Applikation bei stark mit Parasiten befallenen Versuchstieren geprüft. Die angewendeten Dosierungen wurden sehr gut von den Versuchstieren Vertragen.

Die neuen Wirkstoffe können als Anthelmintika sowohl in der Human- als auch in der Veterinärmedizin verwendet werden.

Die neuen Wirkstoffe können in bekannter Weise in die üblichen Formulierungen übergeführt werden.

Die neuen Verbindungen können zusammen mit anderen üblichen Anthelmintika verabreicht werden.

Die neuen Verbindungen können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elixiere, Sirup, Pasten und dergleichen in Betracht. Derartige Träger umfassen feste Verdünnungsmittel oder Füllstoffe, ein steriles, wäßriges Medium sowie verschiedene nicht toxische organische Lösungsmittel und dergleichen. Selbstverständlich können die für eine orale Verabreichung in Betracht kommenden Tabletten und dergleichen mit Süßstoffzusatz und ähnlichen versehen werden. Die therapeutisch wirksame Verbindung soll im vorgenannten Fall in einer Konzentration von etwa 0,5 bis 90 Gew.—% der Gesamtmischung vorhanden sein, d.h. in Mengen, die ausreichend sind, um den obengenannten Dosierungsspielraum zu erreichen.

Die Formulierungen werden in bekannter Weise hergestellt, z.B. durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt: Wasser, nicht toxische organische Lösungsmittel, wie Paraffine (z.B. Erdölfaktionen), pflanzliche Öle (z.B. Erdnuß-(Sesamöl), Alkohole (z.B. Ethylalkohol, Glycerin), Glykole (z.B. Propylenglykol, Polyethylenglykol) und Wasser; feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker); Emulgiermittel, wie nicht ionogene und anionische Emulgatoren (z.B. Polyoxyethylen-Fettsäure-Ester, Polyoxyethylen-Fettalkohol-Ether, Alkylsulfonate und Arylsulfonate), Dispersgiermittel (z.B. Methylcellulose, Stärke und Polyvinylpyrrolidon) und Gleitmittel (z.B. Magnesiumstearat, Talkum, Stearinsäure und Natriumlaurylsulfat).

Im Falle der oralen Anwendung können Tabletten selbstverständlich außer den genannten Trägerstoffen auch Zusätze wie Natriumcitrat, Calciumcarbonat und Dicalciumphosphat, zusammen mit verschiedenen Zuschlagstoffen, wie Stärke, vorzugsweise Kartoffelstärke, Gelatine und dergleichen, enthalten. Weiterhin können Gleitmittel, wie Magnesiumstearat, Natriumlaurylsulfat und Talkum, zum Tablettieren mitverwendet werden.

Im Falle wäßriger Suspensionen und/oder Elixieren, die für die orale Anwendung gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien ein gesetzt werden.

Die Wirkstoff können in Kapseln, Tabletten, Pastillen, Dragees, Ampullen usw. auch in Form von Dosierungseinheiten enthalten sein, wobei jede Dosierungseinheit so angepaßt ist, daß sie eine einzelne Dosis des aktiven Bestandteils liefert.

Die neuen Verbindungen können in den Formulierungen auch in Mischungen mit anderen in der Veterinär- und/oder Humanmedizin zur Behandlung von Infektionen und/oder Erkrankungen benutzten bekannten Wirkstoffen vorliegen, insbesondere L-2,3,5,6-Tetrahydro-6-phenyl-imidazothiazol, Benzimidazolcarbamaten, Praziquantel, Febantel.

Die neuen Wirkstoffe können in üblicher Weise angewendet werden. Die Applikation erfolgt vorzugsweise oral, eine parenterale, insbesondere subkutane, aber auch eine dermale Applikation sind jedoch ebenfalls möglich.

Im allgemeinen hat es sich als vorteilhaft erwiesen, Mengen von etwa 1 bis etwa 100 mg der neuen Verbindungen je kg Körpergewicht pro Tag zur Erzielung wirksamer Ergebnisse zu verabreichen.

Trotzdem kann es gegebenenfalls erforderlich sein, von den genantnen Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt, So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen. Für die Applikation in der Human- und Veterinärmedizin ist der gleiche Dosierungsspielraum vorgesehen. Sinngemaß gelten auch die weiteren obigen Ausführungen.

Beispiel 1

Zu einer Lösung von 26,7 g (0,1 Mol) 3-Methyl-4-(4-trifluormethylthio-phenoxy)-anilin (ölig) und 10,1 g (0,1 Mol)Triethylamin in 300 $cm^3$ Tetrahydrofuran werden unter Rühren bei Raumtemperatur 40,8 g (0,1 Mol) Diiodsalicylsäurechlorid vom Fp.: 94°C — gelöst in Tetrahydrofuran — zugetropft. Danach wird während der Dauer von 3 Stunden bei 60°C nachgerührt, nach dem Abkühlen filtriert, das Lösungsmittel im Vakkum abgezogen, der ölige Rückstand mit Wasser ausgerührt, das Wasser abdekantiert und aus Ligroin umkristallisiert, Fp. des 3,5-Diiod-3'-methyl-4'-(4-trifluormethylthio-phenoxy)-salicylsäureanilid 144°C.

Beispiel 2

Zu einer Lösung von 26,7 g (0,1 Mol) 3-Methyl-4-(4-trifluormethylthio-phenoxy)-anilin (ölig) und 10,1 g Triethylamin in 300 $cm^3$ Tetrahydrofuran werden unter Rühren bei Raumtemperatur 45 g (0,1 Mol) 3,5-Diiodacetylsalicylsäurechlorid vom Fp.: 98°C — gelöst in Tetrahydrofuran — zugetropft. Danach wird 3 Stunden bei 60°C nachgerührt, nach dem Abkühlen filtriert, das Lösemittel im Vakuum abroteirt, der ölige Rückstand mit Wasser ausgezogen und das Wasser abdekantiert. Das 2-Acetoxy-3,5-diiod-3'-methyl-4'-(4-trifluormethylthio-phenoxy)-benzanilid kristallisiert unter wenig Methanol (Fp. 163°C).

10 g dieser Verbindung werden in 100 ml 0,5 n NaOH 1 Stunde bei 60°C gerührt. Danach isoliert man die Verbindung des Beispiels 1.

Analog Beispiel 1 und 2 werden erhalten

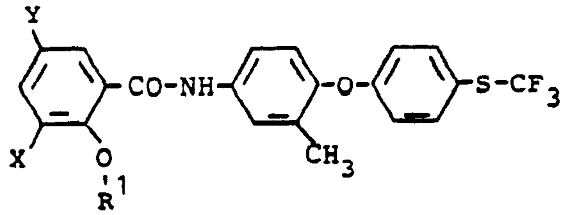

| Bsp. Nr. | $R_1$ | X | Y | Fp (°C) |
|---|---|---|---|---|
| 3 | H | Cl | Cl | 98 |
| 4 | H | Br | Br | 115 |
| 5 | H | Br | Cl | 110 |
| 6 | $COCH_3$ | I | I | 163 |
| 7 | $COCH_3$ | Cl | Cl | 135 |
| 8 | $COCH_3$ | Br | Br | 141 |
| 9 | $COCH_3$ | Br | Cl | 138 |

Beispiel A

Experimentell mit Metacerkarien von Fasciola hepatica infizierte Ratten wurden nach der Infektion oral mittels Schlundsonde behandelt.

a) Ein Teil der Tiere wurde nach der Infektion an drei aufeinanderfolgenden Tagen je einmal behandelt. Die Tiere wurden 2 Wochen nach der Infektion getötet und die Zahl der jugendlichen Leberegel im Leberparenchym bzw. die Zahl der adulten Leberegel im Gallengang bestimmt.

b) Ein Teil der Tiere wurde nach der Infektion einmal behandelt. Die Tiere wurden 12 Wochen nach der Infektion getötet und die Zahl der Leberegel bestimmt.

In der folgenden Tabelle wird angegeben, welche Mindestdosis erforderlich ist um mindestens 95 % Parasitenreduktion im Vergleich zu einer unbehandelten Kontrolle zu erreichen:

| Verbindung | Mindestdosis bei Behandlung | |
|---|---|---|
| | a) | b) |
| 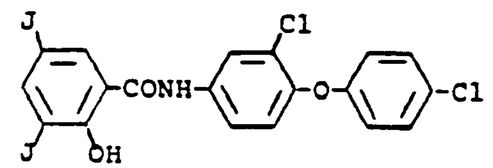 (bekannt aus US–P 3 914 418) | 3 × 100 mg | 25 mg |
| 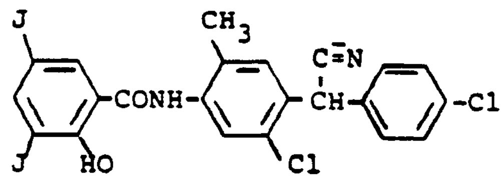 (bekannt aus DE–O 2 610 837) | 3 × 100 mg | 500 mg |
| erfindungsgemäß 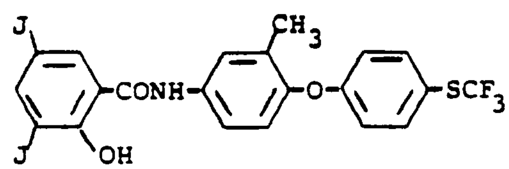 | 3 × 25 mg | 10 mg |

**Patentansprüche**

1. Substituierte Salicylsäureamide der Formel (I)

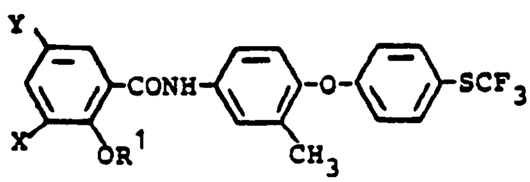

(I)

in der
X und Y für geiche oder verschiedene Halogene stehen und
$R^1$ für Wasserstoff oder den Acetylrest steht.

2. Verfahren zur Herstellung der substituierten Salicylsäureamide der Formel (I)

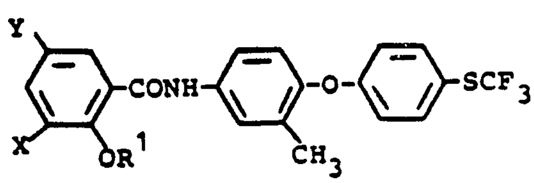

(I)

in der
X und Y für gleiche oder verschiedene Halogene stehen und
$R^1$ für Wasserstoff oder den Acetylrest steht,

dadurch gekennzeichnet, daß man die Chloride der Salicylsäuren oder ihrer Acetylderivate der Formel (II)

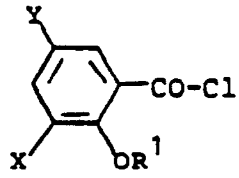

(II)

in der
$R^1$, X und Y die oben angegebene Bedeutung besitzen
mit dem Amin der Formel (III)

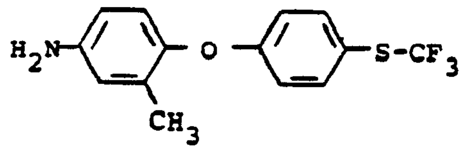

(III)

in Gegenwart eines Verdünnungsmittels, gegebenenfalls in Gegenwart eines Säurebindemittels umsetzt und gegebenenfalls den Acylrest abspaltet.

3. Arzneimittel, gekennzeichnet durch einen Gehalt an mindestens einem substituierten Salicylsäureamid der Formel (I) gemäß Anspruch 1.

4. Anthelmintische Mittel gekennzeichnet durch einen Gehalt an mindestens einem substituierten Salicylsäureamid der Formel (I) gemäß Anspruch 1.

5. Verfahren zur Herstellung von Arzneimitteln, dadurch gekennzeichnet, daß man substituierte Salicylsäureamide der Formel (I) gemäß Anspruch 1 mit Streckmitteln und/oder oberflächenaktiven Mitteln vermischt.

**Claims**

1. Substituted salicylic acid amides of the formula (I)

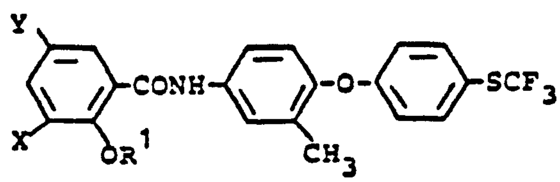

(I)

in which
X and Y represent identical or different halogens and,
$R^1$ represents hydrogen or the acetyl radical.

2. Process for the preparation of the substituted salicylic acid amides of the formula (I)

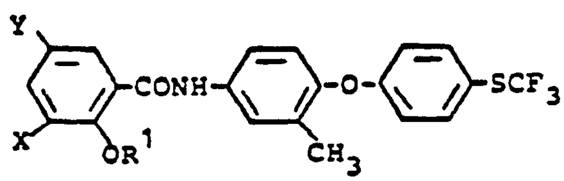

(I)

in which
X and Y represent identical or different hhalogens and
$R^1$ reprsents hydrogen or the acetyl radical, characterised in that the chlorides of the salicylic acids or their acetyl derivatives of the formula (II)

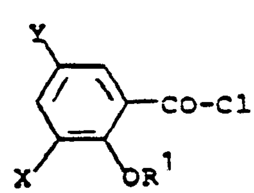

(II)

in which

R', X and Y have the abovementioned meaning, are reacted with the amine of the formula (III)

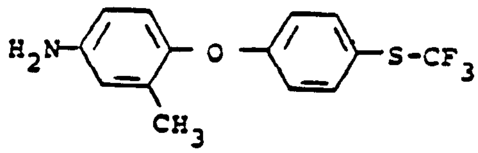

(III)

in the presence of a diluent, if appropriate in the presence of an acid-binding agent, and, if appropriate, the acyl radical is split off.

3. Medicaments, characterised in that they contain at least one substituted salicyclic acid amide of the formula (I) according to Claim 1.

4. Anthelmintic agents, characterised in that they contain at least one substituted salicyclic acid amide of the formula (I) according to Claim 1.

5. Process for the preparation of medicaments, charaterised in that substituted salicylic acid amides of the formula (I) according to Claim 1 are mixed with extenders and/or surface-active agents.

**Revendications**

1. Salicylamides substituées de formule

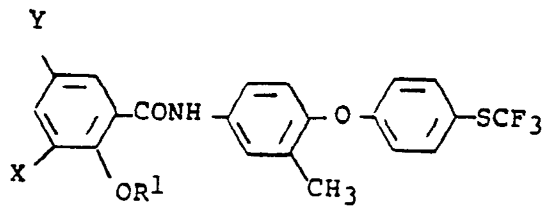

(I)

dans laquelle

X et Y représentent des halogènes identiques ou différents et

R' de l'hydrogène ou le radical acétyle.

2. Procédé de fabrication des salicylamides substituées de formule (I)

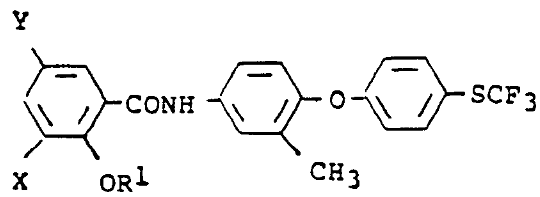

(II)

dans laquelle

X et Y représentent des halogènes identiques ou différents et

R' de l'hydrogène ou le radical acétyle, caractérisé en ce qu'on fait réagir les chlorures des acides salicyliques ou de leurs dérivés acétylés de formule (II)

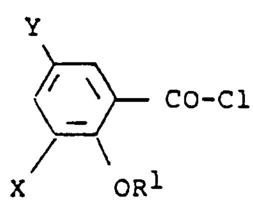

(II)

dans laquelle

R', X et Y possèdent la signification indiquée plus haut,

avec l'amine de formule (III)

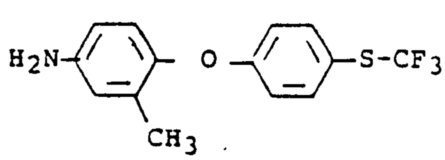

(III)

en présence d'un diluant, éventuellement en présence d'un agent fixateur d'acide, et en ce qu'éventuellement on clive le radical acyle.

3. Médicaments, caractérisés par une teneur en au moins une salicylamide substituée de formule (I) selon la revendication 1.

4. Agent anthelmintiques, caractérisés par une teneur en au moins une salicylamide substituée de formule (I) selon la revendication 1.

5. Procédé de fabrication de médicaments, caractérisé en ce qu'on mélange des salicylamides substituées de formuel (I) selon la revendication 1 avec des diluants et/ou des agents tensioactifs.